# EUROPEAN PATENT SPECIFICATION

(11) **EP 2 421 480 B1**
(45) Date of publication and mention of the grant of the patent: **25.03.2020**
(21) Application number: 10707982.4
(22) Date of filing: 04.02.2010
(51) Int. Cl.: A61F 2/90, A61F 2/954, A61F 2/958, A61F 2/856

(54) **SYSTEM FOR INTRAVASCULAR IMPLANTATION OF A STENT FOR BIFURCATION**
SYSTEM ZUR INTRAVASKULÄREN IMPLANTATION DES STENTS FÜR EINE BIFURKATION
SYSTÈME D'IMPLANTATION INTRAVASCULAIRE D'UNE ENDOPROTHÈSE DE BIFURCATION

(30) Priority: 23.04.2009 PL 38786509
(43) Date of publication of application: 29.02.2012
(73) Proprietor: Balton Sp. z o.o., 00-496 Warszawa (PL)
(72) Inventor: PLOWIECKI, Emil, 03-154 Warszawa (PL); HURKALA, Leszek, 05-120 Legionowo (PL); VASSILEV, Dobrin, Sofia 1784 (BG)
(74) Representative: Orlinska, Dorota Irena
(86) International application number: PCT/PL2010/000011
(87) International publication number: WO 2010/123387

(56) References cited:
- EP-A2- 1 547 546
- WO-A1-00/74595
- WO-A1-01/74273
- US-A1- 2007 203 571
- US-B1- 6 221 090

## Description

Object of the invention is a system for intravascular implantation of a balloon-expandable stent for bifurcation.

The state-of-the art systems for intravascular implantation of stent for bifurcation, utilizing various structural solutions at implantation of the stent, often bring about injuries, especially in bifurcations of arteries. The most frequent problems at implantation of the stent for bifurcation include partial or complete closure of a side branch of artery, contraction of a side branch of artery, dissection at bifurcation of vascules, a decrease in a cross section of a side branch of artery as a result of covering of lumen with struts of an implanted stent, and occurence of thrombosis foci.

To avoid such situations, at present either one stent per each branch is implanted by using separate systems for intravascular implantation for each of the implanted stents or one forked stent, mounted on the system, is implanted.

Another solution is disclosed in the international publication WO 01/74273, wherein a self-expanding stent is in the form of two stent members connected at one side only in a flexible manner.

Still another solution is disclosed in the US patent application US2007203571, wherein a balloon-expandable bifurcation stent comprises a radically expansible support to be positioned in at least a portion of the branch vessel, and a plurality of fronds (struts) extending axially from an end of the support, the fronds configured to be deformably positioned in at least a portion of the main vessel and to apply less radical force to adjacent tissue than the expanded support applies in the branch vessel.

Procedures of intravascular implantation of stents for bifurcation using state-of-the-art methods are extremely complicated to implement and burdened with very high probability of various post-procedural complications in stented arteries- They must be carried out in surgeries provided with very modern equipment. Furthermore, operators carrying out the implantation are required to have high skills and much experience.

Accordingly, there is a demand for new, more effective structural solutions for stents for bifurcation and systems for intravascular implantation of such stents, in order to facilitate implantation at bifurcation place even by less experienced operators and to provide higher safety and efficacy of the procedure.

Aim of the present invention, the object of which is a system for intravascular implantation of a stent for bifurcation, is to increase efficacy and to facilitate implantation procedure, thus increasing safety of procedure and promoting wide use of dedicated stents for bifurcations. In particular, the aims are achieved by a system according to claim 1. Preferred embodiments are subject of dependent claims.

A system for intravascular implantation of a stent for bifurcation, consissof a proximal part and a distal part including an external tube, an internal tube, a profilled balloon and a leading tube, whereas in the distal part of the system, there is a profilled balloon fastened with the proximal part to the external surface of an external tube and with the distal part - to the external surface of an internal tube.

The profilled balloon consists of three inseparable parts: i.e. a distal part - a longer one and of smaller diameter, a proximal part - a shorter one and of greater diameter and a middle part of a specified length, located between the distal part and the proximal part.

Preferably, a ratio of a distal part diameter to a proximal part diameter of the profilled balloon is within the limits of 1.0:1.1 to 1.0:2.0.

Preferably, a length of the middle part of the profilled balloon amounts from 1 to 3 mm.

Preferably, angle θ is within the limits of 15° to 65°.

There are three marker bands, well visible at X-rays, which are fastened on the internal tube of the system for intravascular implantation of the stent for bifurcation, in such a manner that the first marker band coincides with a beginning of long edges of the proximal part of the profilled balloon, the second marker band coincides with a beginning of long edges of the distal part of the profilled balloon, whereas the third marker band coincides with an ending of long edges of the distal part of the profilled balloon.

On the profilled balloon, the stent for bifurcation is appropriately crimped in a detachable manner.

The stent for bifurcation consists of two parts: the distal part, a longer one and having smaller diameter, and the proximal part - a shorter one and having greater diameter, which are connected with connecting struts of a specified length, forming a cell of the stent characterized by an increased surface area.

Preferably, a ratio of the distal part diameter to the proximal part diameter of the stent for bifurcation is within the limits of 1.0:1.1 to 1.0:2.0.

A distance between the distal part and the proximal part of the stent for bifurcation depends on anathomy of the bifurcation place and amounts from 1 to 3 mm.

Preferably, upon detachable crimping of the stent for bifurcation on the profilled balloon, a cell having an increased surface area of the stent for bifurcation coincides with the middle part of the profilled balloon.

The leading tube is fastened, at at least one point, with its proximal part below the profilled balloon to the external tube of the system.

The leading tube is held in a position also by the proximal part of the stent for bifurcation crimped in a detachable manner on the profilled balloon. The distal part of the leading tube extends beyond the proximal part of the stent and is placed along the distal part of the stent.

The distal part of the leading tube has atraumatic ending of a colour different from that of the remaining part of the leading tube.

The system for intravascular implantation of the stent for bifurcation of the invention is characterized by a relatively simple construction and this solution facilitates fast, accurate and thus safe implantation of the stent for bifurcation in vessels.

The solutions are as follows:
- Special and profiled construction of the stent for bifurcation allowing for such positioning of the system for intravascular implantation of the stent for bifurcation during implantation such stent for bifurcation that the distal part of the stent for bifurcation is positioned beyond a fork of a vessel in a narrower part of the vessel, the proximal part of the stent for bifurcation is positioned before the fork, and the middle part of the stent, with connecting struts of the stent, forming at the same time a cell of the stent having a special, increased surface area, is positioned exactly at the lumen of the side branch while not disturbing the blood flow through the side branch of the fork.
- Locating three marker bands, well visible at X-rays, on the internal tube to ensure accurate positioning and visibility of the stent for bifurcation during its implantation in vessels.
- The stent for bifurcation crimped in a detachable manner on the profilled balloon in such a way that the cell of an increased surface area of the stent for bifurcation coincides with the middle part of the profilled balloon
- A special shape of the profilled balloon that prevents from shifting the carina tip during implantation of the stent for bifurcation, thereby preventing from blocking blood flow through the side branch of artery.
- Designation of the distal end of the leading tube with a different colour from that of its remaining part, a way of its fastening to the external tube of the system for intravascular implantation of the stent for bifurcation, and also fastening of the leading tube through the proximal part of the stent for bifurcation, facilitate introduction and removal of the system for intravascular implantation of the stent for bifurcation from blood vessels upon implanting the stent for bifurcation.

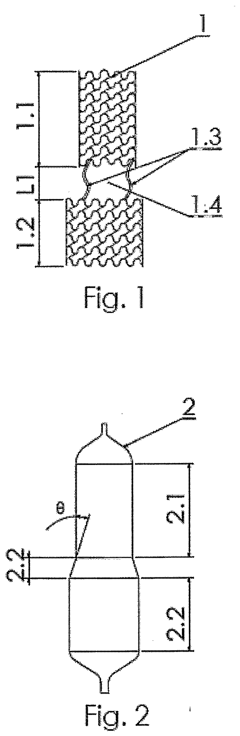

The stent for bifurcation is presented on Fig.1, which shows a view of the stent for bifurcation with a depicted cell of the stent having an increased surface area **1.4.**

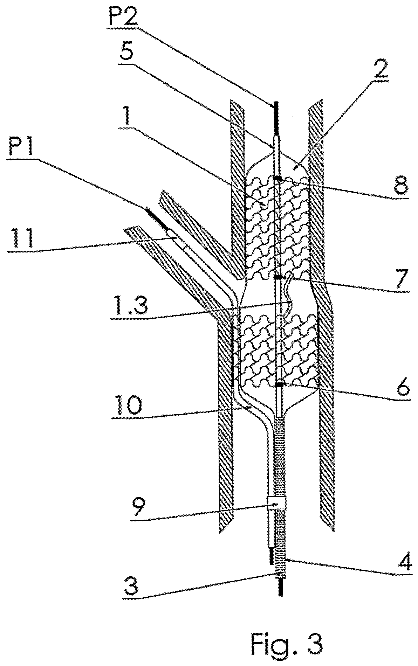

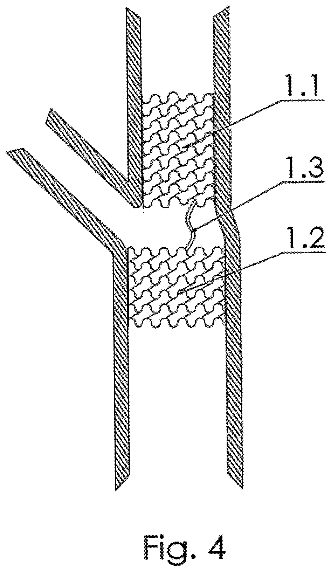

The system for intravascular implantation of the stent for bifurcation in an embodiment is presented in a drawing, in which Fig.2, shows a view of the profilled balloon, Fig.3 shows a view of the distal part of the system for intravascular implantation of the stent for bifurcation and its location in a fork of artery, being a place of implantation of the stent, Fig.4 shows arrangement of the stent for bifurcation upon its implanting in an artery and in its main branch.

The stent for bifurcation **1** consists of the distal part, that is longer and has a smaller diameter **1.1,** and the proximal part - that is shorter and has a greater diameter **1.2,** connected with connecting struts **1.3** having length **L1**, forming a cell of the stent of an increased surface area **1.4.**

The system for intravascular implantation of the stent for bifurcation of the invention in its distal part **3** consists of the profilled balloon **2** fastened to the system **3** in such a way that the proximal part **2.2** of the profilled balloon **2** is fastened to the external surface of an external tube **4** of the system **3,** and the distal part **2.1** of the profilled balloon **2** is fastened to the external surface of the internal tube **5** of the system **3.**

The profilled balloon **2** consists of three inseparable parts:
a distal part - that is longer and has smaller diameter **2.1,** proximal - that is shorter and has greater diameter **2.2** and of a middle part **2.3** having a specified length, located between the distal part **2.1** and the proximal part **2.2.** Between long edges of the distal part **2.1** and an edge of the middle part **2.3** of the profilled balloon **2,** there is an angle **Θ.** The stent for bifurcation **1** is crimped in a detachable manner on the profilled balloon **2,** in such a way that the cell of the stent of an increased surface area **1.4** of the stent for bifurcation **1** coincides with the middle part **2.3** of the profilled balloon **2.** The leading tube **10** is fastened at at least one point **9** to the external tube **4** of the system **3** below the profilled balloon **2,** the distal part **11** of the leading tube **10** having an atraumatic ending and colour different from that of the remaining part of the leading tube **10.** The leading tube **10** is held by the proximal part **1.2** of the stent for bifurcation **1** crimped in a detachable manner on the profilled balloon **2.** The distal part **11** of the leading tube extends beyond the proximal part **1.2** of the stent for bifurcation **1** and is placed along the distal part **1.1** of the stent for bifurcation **1.**

Three marker bands **6, 7, 8,** visible at X-rays, are fastened on the internal tube **5** of the system for intravascular implantation of the stent for bifurcation **3** in such a way that the marker band **6** coincides with a beginning of long edges of the proximal part **2.2** of the profilled balloon **2,** the marker band **7** coincides with a beginning of long edges of the distal part **2.1** of the profilled balloon **2,** whereas the marker band **8** coincides with the ending of the long edges of the distal part **2.1** of the profilled balloon **2.**

The system for intravascular implantation of the stent **3** of the invention is introduced into body in such a way, that two guide wires are advanced to the bifurcation site, the first P1 of which is located in a side branch of artery, the second P2 - in the main branch of artery.

Then, on the guide wires P1 and P2, the system for intravascular implantation of the stent **3** of the invention is introduced.

The leading tube **10** fastened to the external tube **4** is put onto the first guide wire P1. The internal tube **5** of the system for intravascular implantation of the stent **3** is put onto the second guide wire P2.

Then, moving on the guide wires P1 and P2 the whole system for intravascular implantation of the stent **3,** while controlling at the same time a position of the three marker bands **6, 7, 8,** at X-rays, the stent for bifurcation **1** is very precisely placed with the profilled balloon **2** at the bifurcation site: the leading tube **10** on the guide wire P1 enters a side branch of the artery, and the remaining part of the system **3** is introduced on the guide P2 into the main branch of the artery.

Such introduction of the system for intravascular implantation of the stent for bifurcation **3** results in that the distal part **1.1** of the stent for bifurcation is situated beyond the bifurcation place of the artery, in the main branch, the proximal part **1.2** of the stent for bifurcation - before the bifurcation place, and the middle part of the stent, including the cell of the stent having a special, increased surface area **1.4,** is located in the lumen of the side branch.

Liquid inflation under pressure 6 to 16 atm expanding the profilled balloon **2.** Thereby opening of the stent for bifurcation **1.** Fully opened stent for bifurcation **1** increasing lumen diameter of the vessel, thus eliminating stenosis.

Upon complete extension of the stent for bifurcation **1** its middle part, including the cell of the stent having a special, increased surface area **1.4,** is located exactly in lumen of the side branch, which enables free blood flow through the side branch of artery.

Then, the liquid is removed from the profilled balloon **2,** the whole system **3** with an deflated profilled balloon **2** and the leading tube **10** is withdrawn from blood circulation system. At the end, the guide wires P1 and P2 are removed. The opened stent for bifurcation **1** remains in a place where stenosis of artery existed, while preventing vessel from renarrowing.

## Claims

1. A system for intravascular implantation of a balloon-expandable stent for bifurcation, **characterized in that**
the balloon-expandable stent for bifurcation (1) consists of two tubular mesh parts: a distal part (1.1) that has a smaller diameter and a proximal part (1.2) that has a greater diameter, whereas the distal part (1.1) and the proximal part (1.2) are connected with two connecting struts (1.3) forming a cell of the stent of an increased surface area (1.4) as compared to cells of the two tubular mesh parts (1.1, 1.2), wherein the connecting struts (1.3) have length (L1) from 1 to 3 mm and a distance between the distal part (1.1) and the proximal part (1.2) of the stent for bifurcation amounts from 1 to 3 mm, and wherein
the system for intravascular implantation (3), consists of a proximal part and a distal part, and includes an external tube (4), an internal tube (5) and a profilled balloon (2), whereas in the distal part of the system (3), on the internal tube (5) marked with three marker bands (6, 7, 8), visible at X-rays, there is positioned the profilled balloon (2) with a proximal part (2.2) fastened to the external surface of the external tube (4), and with a distal part (2.1) fastened to the external surface of the internal tube (5), wherein the stent for bifurcation (1) is crimped on the profilled balloon (2) in a detachable manner, and wherein
the profilled balloon (2) consists of three inseparable parts: the distal part (2.1) that has a smaller diameter, the proximal part (2.2) that has a greater diameter, and the middle part (2.3) having a length from 1 to 3 mm located between the distal part (2.1) and the proximal part (2.2), wherein upon detachable crimping of the stent for bifurcation (1) on the profilled balloon (2), the cell of an increased surface area (1.4) of the stent for bifurcation (1), coincides with the middle part (2.3) of the profilled balloon (2).

2. The system of Claim 1, **characterized in that** a leading tube (10) is fastened with its proximal part at at least one point (9) to the external tube (4) below the profilled balloon (2), whereas a distal part (11) of the leading tube (10) is ended atraumatically and marked with a different colour than that of the remaining part of the leading tube (10).

3. The system of Claim 1 or 2, **characterized in that** the ratio of the diameter of the distal part (2.1) to the diameter of the proximal part (2.2) of the profilled balloon (2) is within the range from 1.0:1.1 to 1.0:2.0.

4. The system of any one of Claims 1 to 3, **characterized in that** an angle θ formed between a long edges of the distal part (2.1) of the profilled balloon (2) and anedge of the middle part (2.3) of the profilled balloon (2) is within the limits from 15° to 65° .

5. The system of any one of Claims 2 to 4, **characterized in that** the leading tube (10) is additionally held by the proximal part (1.2) of the stent for bifurcation (1) crimped in a detachable manner on the profilled balloon (2).

6. The system of Claim 1, **characterized in that** the ratio of the diameter of the distal part (1.1) to the diameter of the proximal part (1.2) of the stent for bifurcation is within the limits of 1.0:1.1 to 1.0:2.0.

## Patentansprüche

1. Ein System zur intravaskulären Implantation eines ballonexpandierbaren Stents zur Verzweigung, **dadurch gekennzeichnet, dass**
der ballonexpandierbare Stent zur Verzweigung (1) aus zwei rohrförmigen Netzteilen besteht: einem distalen Teil (1.1) mit einem kleineren Durchmesser und einem proximalen Teil (1.2) mit einem größeren Durchmesser, während der distale Teil (1.1) und der proximale Teil (1.2) mit zwei Verbindungsstreben (1.3) verbunden sind, die eine Zelle des Stents mit einer vergrößerten Oberfläche (1.4) im Vergleich zu den Zellen der beiden rohrförmigen Netzteile (1.1, 1.2) bilden, wobei die Verbindungsstreben (1.3) eine Länge (L1) von 1 bis 3 mm und einen Abstand zwischen dem distalen Teil (1.1) und dem proximalen Teil (1.2) des Stents zur Verzweigung von 1 bis 3 mm aufweisen, und worin
das System zur intravaskulären Implantation (3), bestehend aus einem proximalen Teil und einem distalen Teil, ein Außenrohr (4), ein Innenrohr (5) und einen profilierten Ballon (2) beinhaltet, während im distalen Teil des Systems (3) auf dem Innenrohr (5), das mit drei Markierungsbändern (6, 7, 8) markiert ist, die in Röntgenstrahlen sichtbar sind, der Profilballon (2) mit einem proximalen Teil (2.2), der an der Außenfläche des Außenrohrs (4) befestigt ist, und mit einem distalen Teil (2.1), der an der Außenfläche des Innenrohrs (5) befestigt ist, positioniert ist, worin der Stent zur Verzweigung (1) auf den Profilballon (2) lösbar gecrimpt ist, und worin
der profilierte Ballon (2) aus drei untrennbaren Teilen besteht: wobei der distale Teil (2.1) einen kleineren Durchmesser aufweist, der proximale Teil (2.2) einen größeren Durchmesser aufweist und der mittlere Teil (2.3) eine Länge von 1 bis 3 mm aufweist, die zwischen dem distalen Teil (2.1) und dem proximalen Teil (2.2) angeordnet ist, wobei beim lösbaren Crimpen des Stents zur Verzweigung (1) auf dem profilierten Ballon (2) die Zelle mit einer vergrößerten Oberfläche (1.4) des Stents zur Verzweigung (1) mit dem Mittelteil (2.3) des profilierten Ballons (2) zusammenfällt.

2. Das System nach Anspruch 1, **dadurch gekennzeichnet, dass** ein Führungsrohr (10) mit seinem proximalen Teil an mindestens einem Punkt (9) am Außenrohr (4) unterhalb des profilierten Ballons (2) befestigt ist, während ein distaler Teil (11) des Führungsrohrs (10) atraumatisch beendet und mit einer anderen Farbe als der des restlichen Teils des Führungsrohrs (10) markiert ist.

3. Das System nach Anspruch 1 oder 2, **dadurch gekennzeichnet, dass** das Verhältnis des Durchmessers des distalen Teils (2.1) zum Durchmesser des proximalen Teils (2.2) des profilierten Ballons (2) innerhalb des Bereichs von 1,0:1,1 bis 1,0:2,0 liegt.

4. Das System nach einem der Ansprüche 1 bis 3, **dadurch gekennzeichnet, dass** ein Winkel θ, der zwischen einer Längskante des distalen Teils (2.1) des Profilballons (2) und einer Kante des mittleren Teils (2.3) des Profilballons (2) gebildet ist, innerhalb der Grenzen von 15° bis 65° liegt.

5. Das System nach einem der Ansprüche 2 bis 4, **dadurch gekennzeichnet, dass** das Führungsrohr (10) zusätzlich vom proximalen Teil (1.2) des Stents zur Verzweigung (1) gehalten wird, der am profilierten Ballon (2) lösbar gecrimpt ist.

6. Das System nach Anspruch 1, **dadurch gekennzeichnet, dass** das Verhältnis des Durchmessers des distalen Teils (1.1) zum Durchmesser des proximalen Teils (1.2) des Stents zur Verzweigung innerhalb der Grenzen von 1,0:1,1 bis 1,0:2,0 liegt.

## Revendications

1. Un système d'implantation intravasculaire d'une endoprothèse expansible par ballonnet pour la bifurcation, **caractérisé en ce que**
l'endoprothèse expansible par ballonnet pour la bifurcation (1) se compose de deux parties tubulaires en maille: une partie distale (1.1) qui a un plus petit diamètre et une partie proximale (1.2) qui a un plus grand diamètre, tandis que la partie distale (1.1) et la partie proximale (1.2) sont reliées avec deux entretoises de connexion (1.3) formant une cellule de l'endoprothèse d'une surface augmentée (1.4) par rapport aux cellules des deux parties tubulaires en maille (1.1, 1.2), dans lequel les entretoises de connexion (1.3) ont une longueur (L1) de 1 à 3 mm et une distance entre la partie distale (1.1) et la partie proximale (1.2) de l'endoprothèse pour la bifurcation est de 1 à 3 mm, et dans lequel
le système d'implantation intravasculaire (3), se compose d'une partie proximale et d'une partie distale, et comprend un tube externe (4), un tube interne (5) et un ballonnet profilé (2), tandis que dans la partie distale du système (3), sur le tube interne (5) marqué de trois bandes de repérage (6, 7, 8), visibles aux rayons X, se positionne le ballonnet profilé (2) avec une partie proximale (2.2) fixée à la surface externe du tube externe (4), et avec une partie distale (2.1) fixée à la surface externe du tube interne (5), dans lequel l'endoprothèse pour la bifurcation (1) est serti sur le ballonnet profilé (2) d'une manière détachable, et dans lequel
le ballonnet profilé (2) se compose de trois parties inséparables : la partie distale (2.1) qui a un plus petit diamètre, la partie proximale (2.2) qui a un plus grand diamètre, et la partie centrale (2.3) ayant une longueur de 1 à 3 mm située entre la partie distale (2.1) et la partie proximale (2.2), dans lequel, lors du sertissage détachable de l'endoprothèse pour la bifurcation (1) sur le ballonnet profilé (2), la cellule d'une surface augmentée (1.4) de l'endoprothèse pour la bifurcation (1) coïncide avec la partie centrale (2.3) du ballonnet profilé (2).

2. Le système selon la revendication 1, **caractérisé en ce qu'**un tube avant (10) est fixé avec sa partie proximale en au moins un point (9) au tube externe (4) en dessous du ballonnet profilé (2), tandis qu'une partie distale (11) du tube avant (10) est terminée de façon atraumatique et marquée d'une couleur différente de celle de la partie restante du tube avant (10).

3. Le système selon la revendication 1 ou 2, **caractérisé en ce que** le rapport du diamètre de la partie distale (2.1) au diamètre de la partie proximale (2.2) du ballonnet profilé (2) est dans la plage de 1,0:1,1 à 1,0:2.0.

4. Le système selon l'une quelconque des revendications 1 à 3, **caractérisé en ce qu'**un angle θ formé entre un bord long de la partie distale (2.1) du ballonnet profilé (2) et un bord de la partie centrale (2.3) du ballonnet profilé (2) est dans les limites de 15° à 65°.

5. Le système selon l'une quelconque des revendications 2 à 4, **caractérisé en ce que** le tube avant (10) est en outre maintenu par la partie proximale (1.2) de l'endoprothèse pour la bifurcation (1) serti de manière détachable sur le ballonnet profilé (2).

6. Le système selon la revendication 1, **caractérisé en ce que** le rapport du diamètre de la partie distale (1.1) au diamètre de la partie proximale (1.2) de l'endoprothèse pour la bifurcation est dans les limites de 1,0:1,1 à 1,0:2,0.
